# EUROPEAN PATENT APPLICATION

(11) **EP 4 151 207 A1**
(43) Date of publication of application: **22.03.2023**
(21) Application number: 22184666.0
(22) Date of filing: 13.07.2022
(51) Int. Cl.: A61K 31/136, A61K 31/4184, A61K 31/427, A61K 31/519, A61K 31/555, A61K 31/675, A61K 31/704, A61K 39/00, A61K 45/06, A61P 35/00

(54) **BENZIMIDAZOLES FOR USE IN THE TREATMENT OF CONDITIONS INVOLVING CD47 UPREGULATION OR FOR INCREASING PHAGOCYTOSIS OF A CELL**

(30) Priority: 17.09.2021 US 202163245540 P
(71) Applicant: National Health Research Institutes, Miaoli County 35053 (TW); Academia Sinica, Taipei, Taiwan (TW)
(72) Inventor: Chen, Chih-Hao, Taipei City (TW); Yen, Wan-Ching, California (US); Yeh, Teng-Kuang, Taipei, (TW); Wang, Hwei-Jiung, Taipei City, (TW); Huang, Kai-Fa, New Taipei City (TW)
(74) Representative: Purdylucey Intellectual Property

(57) **Abstract**

Disclosed are compounds of formula (I) for use in methods for treatments of conditions involving CD47 upregulation or for increasing phagocytosis of a cell: 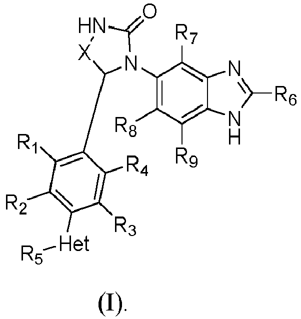 Variables R₁-R₉, X, and Het are defined therein. Also disclosed are pharmaceutical compositions each containing such a compound and an anti-cancer agent.

## Description

### BACKGROUND

CD47 is a protein expressed on normal cells serving as an immune checkpoint to inhibit macrophage-mediated destruction of its host cells. It binds to signal regulatory protein α (SIRPα), a membrane glycoprotein expressed on myeloid cells. The CD47-SIRPα binding forms a signaling axis marking the host cell as self to avoid phagocytosis by the immune system.

CD47 is often upregulated in cancer cells, forming abundant CD47-SIRPα axes for cancer cells to escape elimination by the body. Further, CD47 upregulation is also found in patients having a fibrotic disease, atherosclerosis, or an infectious disease. See, e.g., Cui et al., Nat. Commun. 11, 2795 (2020); Kojima, et al., Nature 536, 86-90 (2016); and Tal et al., mBio 11 (2020). Blocking the CD47-SIRPα signaling axis promotes destruction of cancer cells and provides novel treatments for the fibrotic disease, atherosclerosis, and the infectious disease.

Nevertheless, it is a challenge to develop an effective treatment of a condition involving CD47 upregulation via inhibiting the CD47-SIRPα signaling axis as there is not clear guidance provided by publications as to how to select compounds for this treatment.

There is a need to develop an effective method of treating a condition involving CD47 upregulation.

### SUMMARY

The present invention is based on an unexpected discovery that certain benzimidazole compounds are useful to treat a condition (e.g., cancer) involving CD47 upregulation.

In one aspect, this invention relates to a compound for use in a method for treating a condition involving CD47 upregulation. The compound is a benzimidazole compound of formula (I): 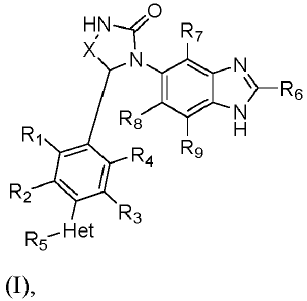 in which, each of R₁, R₂, R₃, R₄, R₆, R₇, R₈, and R₉, independently, is H, halo, or C₁₋₆alkyl; R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl; Het is a C₅₋₆ heteroaryl; and X is CH₂ or CO.

A compound of formula (I) can be used as a stereoisomer or a mixture of stereoisomers thereof, or a pharmaceutically acceptable salt, solvate, hydrate, co-crystal, clathrate, or polymorph thereof.

Compounds of formula (I) can have one or more of the following features:
(i) each of R₁, R₂, R₃, and R₄, independently, is H or F; (ii) each of R₆, R₇, R₈, and R₉ is H; (iii) R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl; and (iv) Het is a moiety of tetrazole, oxadiazole, thiazole, isoxazole, or thiophene.

Exemplary compounds of formula (I) include Compounds 1-35, the structures of which are shown below:

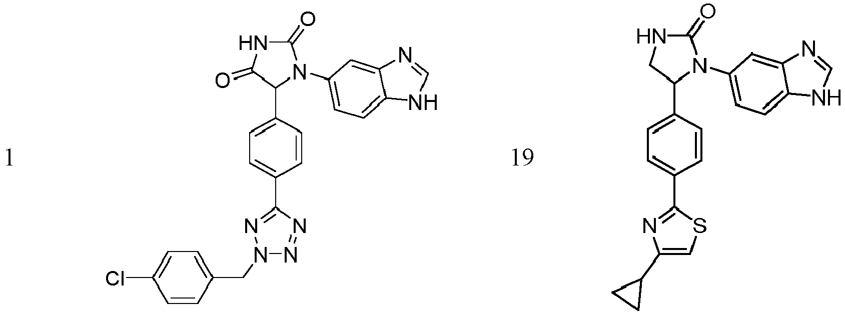

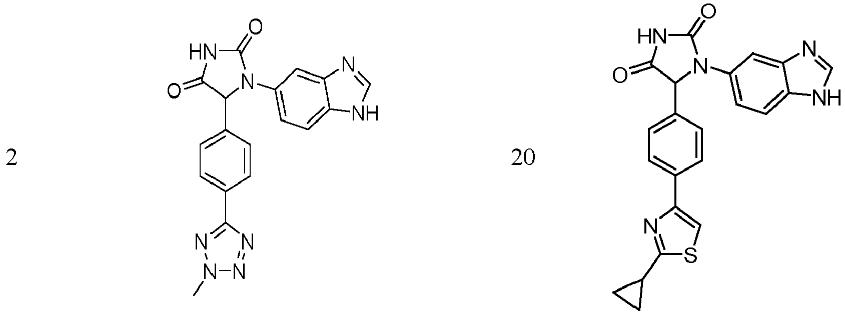

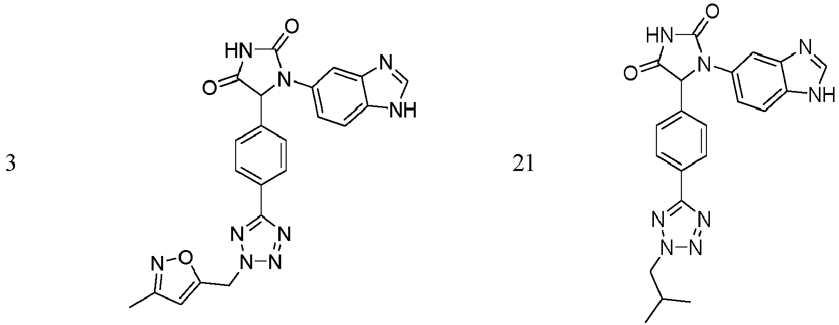

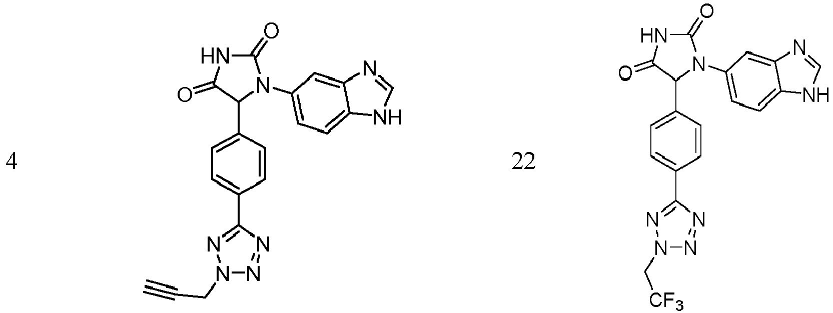

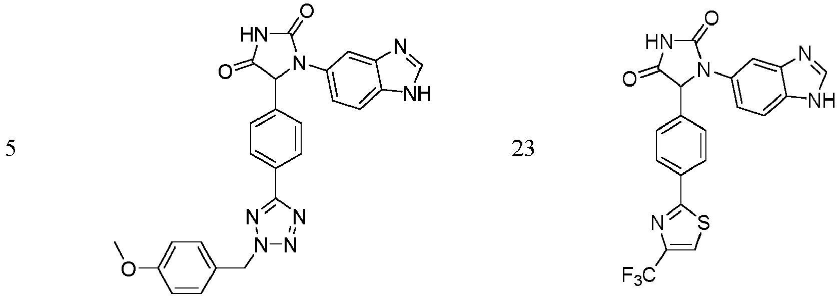

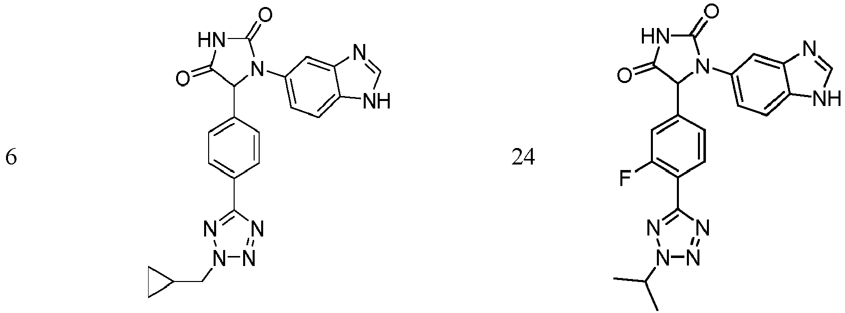

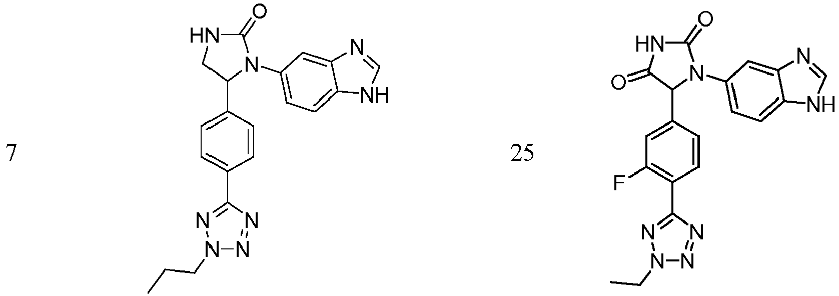

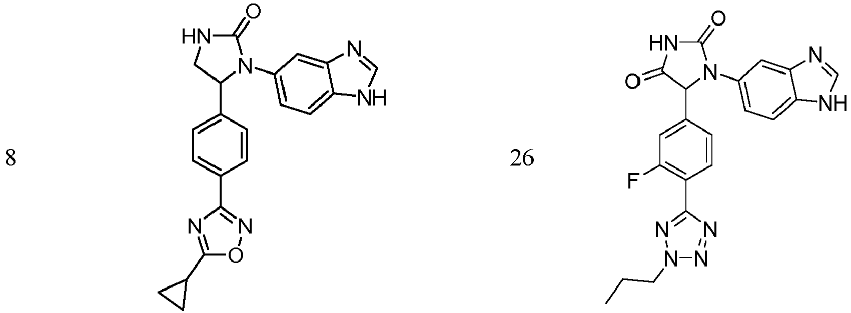

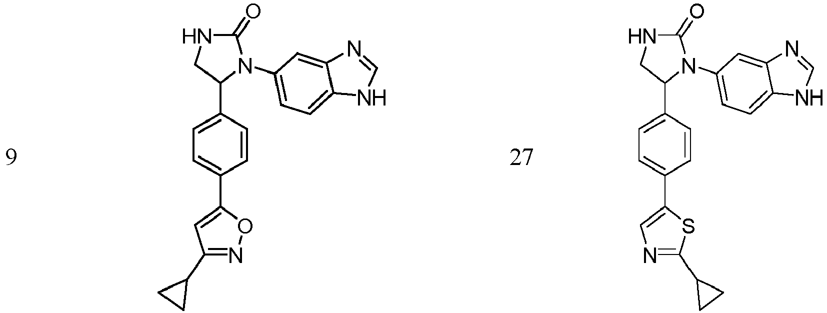

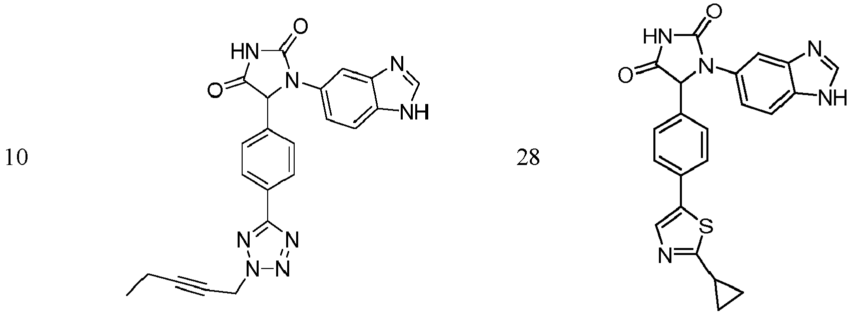

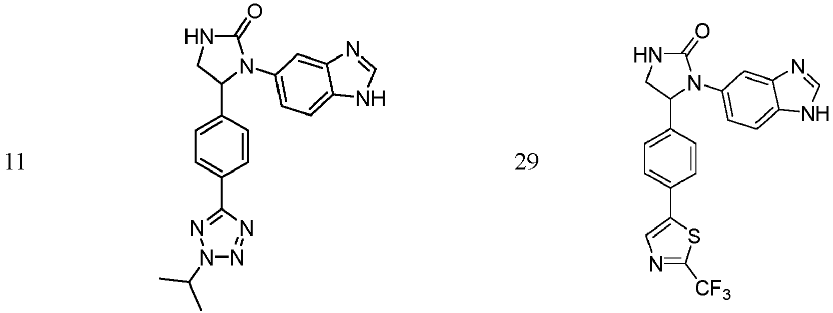

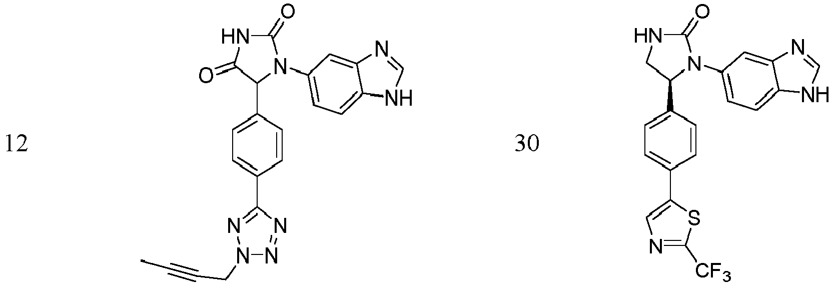

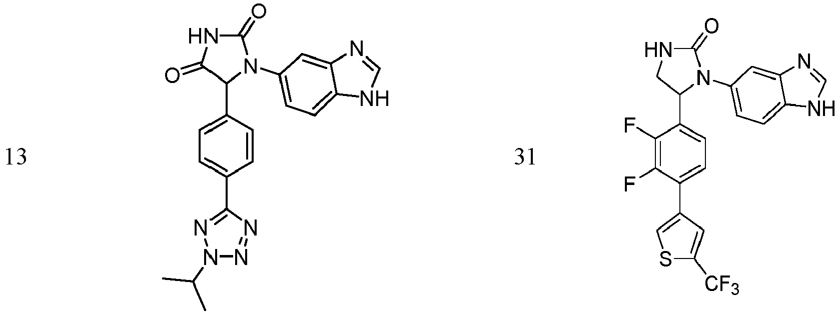

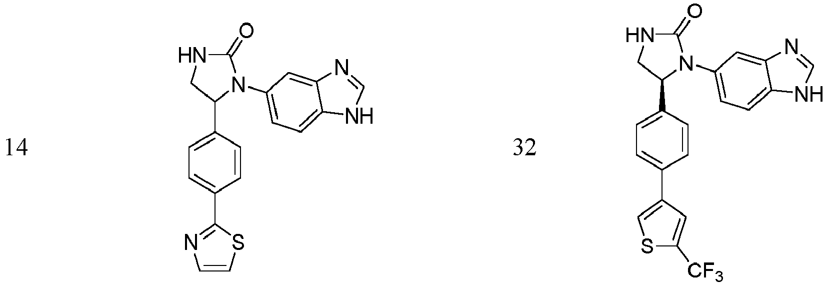

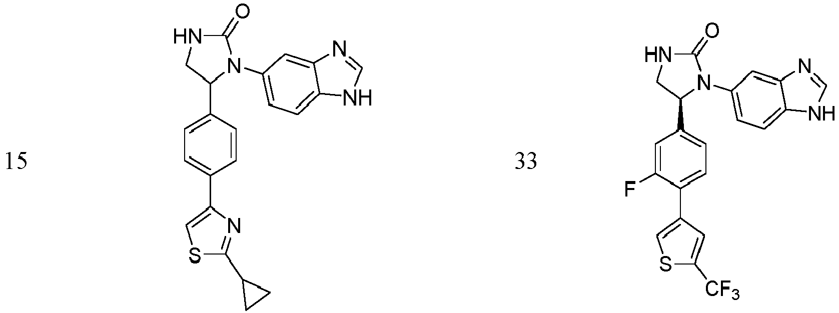

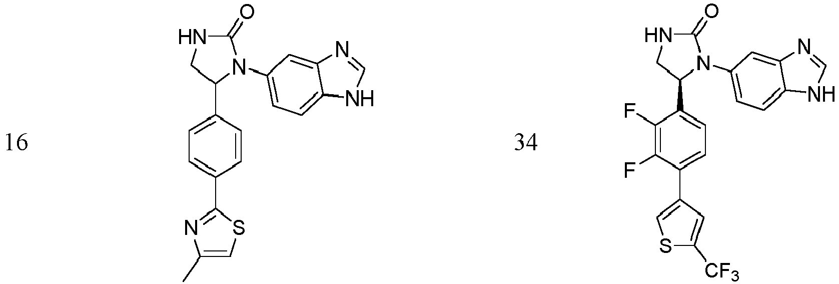

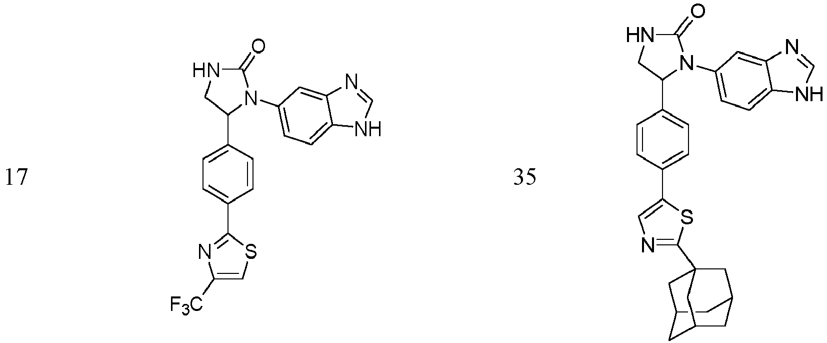

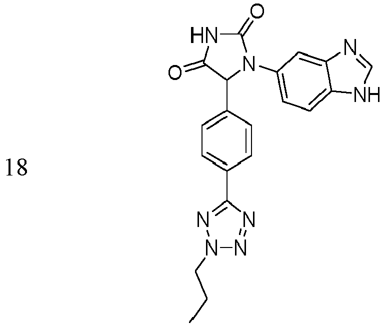

Preferred compounds are Compound 30, Compound 32, and Compound 34.

The compound is typically formulated into a pharmaceutical composition so that it can be conveniently administered to a patient in need of treatment at an effective amount (e.g., at a dosage of 10 mg/kg to 200 mg/kg per day).

The condition involving CD47 upregulation includes a cancer, a fibrotic disease, atherosclerosis, or an infectious disease.

Cancers treatable by a compound of formula (I) include cancers of the ovary, breast, colon, bladder, prostate, brain, head-and-neck, lung, stomach, pancreas, non-Hodgkin's lymphoma, chronic myeloid leukemia in blast crisis, acute lymphoblastic leukemia, and multiple myeloma. In addition to a compound of formula (I), a patient is often also administered with an anti-cancer agent selected from the group consisting of a therapeutic antibody, a molecular targeting agent, a chemotherapeutic agent, an immuno-therapeutic agent, and combinations thereof. Useful therapeutic antibodies include an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and any combinations thereof. Specific antibodies are CD47 antibodies B6H12.2, Hu5F9-G4, IT-061, CC-90002, SRF231, SHR-1603, AO-176, TJC4, TJC4-CK, SY102, PSTx-23, MBT-001, IMC-002, HMBD-004B, HLX24, BAT6004, AUR-105, AUR-104, and LYN00301; CD47 bi-specific antibodies NI-1701, IBI188, IMM03, NI-1801, PDL1/CD47 BsAv, IMM2502, IBI322, ABP-160, HMBD-004A, and BH-29xx; SIRPα inhibitors ALX148, KWAR23, TTI-621, TTI-622, OSE-172, CC-95251, IMM01, FSI-189, and CTX-5861; and SIRPα bi-specific inhibitors SL-172154, IMM02, and DSP107.

Examples of chemotherapeutic agents are those selected from the group consisting of doxorubicin, epirubicin, idarubicin, methotrexate, mitoxantrone, oxaliplatin, cyclophosphamide, and combinations thereof. Molecular targeting agents include gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof. Immuno-therapeutic agents can be selected from the group consisting of nivolumab, ipilimumab, pembrolizumab, atezolizumab, avelumab, durvalumab, and combinations thereof.

Another condition treatable by a compound of formula (I) is a fibrotic disease, e.g., bladder fibrosis, heart fibrosis, idiopathic pulmonary fibrosis, kidney fibrosis, lung fibrosis, liver fibrosis, myelofibrosis, pancreas fibrosis, and scleroderma.

An infectious disease can also be treated by a compound of formula (I), e.g., those caused by a virus, a bacterium, or a protozoan.

Another aspect of this method relates to a compound of formula (I) for increasing phagocytosis of a cell, comprising contacting the cell with a compound of formula (I): 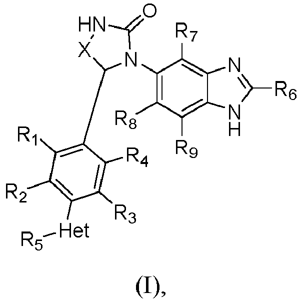 and exposing the cell to a phagocytic cell, wherein the cell expresses CD47.

R₁, R₂, R₃, R₄, R₅, R₆, R₇, R₈, and R₉, Het, and X are defined above.

The cell can be a cancer cell in the ovary, breast, colon, bladder, prostate, brain, head-and-neck, lung, stomach, pancreas, lymphatic system, bone, or blood. The phagocytic cell is typically a myeloid cell (e.g., a macrophage, a monocyte, a neutrophil, a basophil, an eosinophil, and a dendritic cell) having signal regulatory protein alpha on its surface.

A compound of formula (I) can be used alone or in combination with an anti-cancer agent selected from the group consisting of a therapeutic antibody, a molecular targeting agent, a chemotherapeutic agent, an immuno-therapeutic agent, and combinations thereof. Any of the anti-cancer agent described above can be used in this method. In addition, the method can contain a step of contacting the cell with the above-described CD47 inhibitor, the above-described SIRPα inhibitor, or any combination thereof.

Also within the scope of this invention is a pharmaceutical composition comprising an anti-cancer agent and a compound of formula (I):

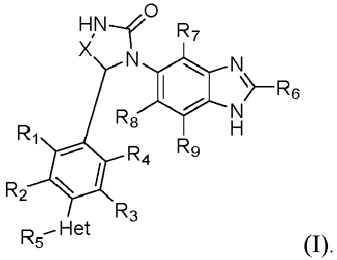

R₁, R₂, R₃, R₄, R₅, R₆, R₇, R₈, and R₉, Het, X, and the anti-cancer agent are defined above.

The term "alkyl" herein refers to a straight or branched hydrocarbon group, containing 1-20 (e.g., 1-10 and 1-6) carbon atoms. Exemplary alkyl groups are methyl ("Me"), ethyl ("Et"), n-propyl, iso-propyl, n-butyl, iso-butyl, and tert-butyl. Alkyl includes its halo substituted derivatives, i.e., haloalkyl, which refers to alkyl substituted with one or more halogen (chloro, fluoro, bromo, or iodo) atoms. Examples include trifluoromethyl, bromomethyl, and 4,4,4-trifluorobutyl. The term "alkoxy" refers to an -O-alkyl group (e.g., methoxy, ethoxy, propoxy, and isopropoxy). Alkoxy includes haloalkoxy, namely, alkoxy substituted with one or more halogen atoms, e.g., -O-CH₂Cl and -O-CHClCH₂Cl.

The term "cycloalkyl" refers to a saturated and partially unsaturated monocyclic, bicyclic, tricyclic, or tetracyclic hydrocarbon group having 3 to 12 carbons (e.g., C₃₋₁₀). Examples are cyclopropyl, cyclobutyl, cyclopentyl, cyclopentenyl, cyclohexyl, cyclohexenyl, cycloheptyl, and cyclooctyl. The term "cycloalkyloxy" refers to an -O-cycloalkyl group, e.g., cyclohexyloxy. Cycloalkyloxy includes halocycloalkyloxy, referring to cycloalkyloxy substituted with one or more halogen atoms.

The term "heterocycloalkyl" refers to a nonaromatic 3-8 membered monocyclic, 8-12 membered bicyclic, or 11-14 membered tricyclic ring system having one or more heteroatoms (e.g., O, N, P, and S). Examples of heterocycloalkyl groups include piperazinyl, piperidinyl, imidazolidinyl, azepanyl, pyrrolidinyl, dihydrothiadiazolyl, dioxanyl, morpholinyl, tetrahydropuranyl, and tetrahydrofuranyl. The term "heterocycloalkyloxy" refers to an -O-heterocycloalkyloxy. Each of hetercycloalkyl and heterocycloalkyloxy include its halogenated versions, i.e., those having one or more substitutions of halogen atoms.

The term "alkenyl" refers to a straight or branched, monovalent, unsaturated aliphatic chain having 2 to 20 carbon atoms (e.g., C2-4, C2-6, and C2-10) and one or more carbon-carbon double bonds. Examples are ethenyl (also known as vinyl), 1-methylethenyl, 1-methyl-1- propenyl, 1-butenyl, 1-hexenyl, 2-methyl-2-propenyl, 1-propenyl, 2-propenyl, 2-butenyl, and 2- pentenyl.

The term "alkynyl" refers to a straight or branched aliphatic chain having 2 to 20 carbon atoms (e.g., C2-4, C2-6, and C2-10) and one or more carbon-carbon triple bonds. Examples are ethynyl, 2-propynyl, 2-butynyl, 3-methylbutnyl, and 1-pentynyl.

The term "aryl" refers to a monovalent or bivalent, 6-carbon monocyclic, 10-carbon bicyclic, 14-carbon tricyclic aromatic ring system wherein each ring can have 1 to 5 substituents. Examples include phenyl, naphthyl, and anthracenyl. The term "aralkyl" refers to alkyl substituted with an aryl group. The term "aryloxy" refers to an -O-aryl group, e.g., phenoxy.

The term "heteroaryl" refers to a monovalent or bivalent, aromatic 5-8 membered monocyclic, 8-12 membered bicyclic, or 11-14 membered tricyclic ring system having one or more heteroatoms (e.g., O, N, P, and S). Examples include triazolyl, oxazolyl, thiadiazolyl, tetrazolyl, oxadiazolyl, oxazolyl, isoxazolyl, pyrazolyl, pyridyl, carbazolyl, tetrahydropyranyl, furyl, imidazolyl, benzimidazolyl, pyrimidinyl, thienyl, quinolinyl, indolyl, thiazolyl, and benzothiazolyl. The term "heteroaralkyl" refers to an alkyl group substituted with a heteroaryl group. The term "heteroaryloxy" refers to an -O-heteroaryl group.

The terms "halo" refers to a fluoro, chloro, bromo, or iodo radical. The term "amino" refers to a radical derived from amine, which is unsubstituted or mono-/disubstituted with alkyl, aryl, cycloalkyl, heterocycloalkyl, or heteroaryl. The term "alkylamino" refers to alkyl-NH-. The term "dialkylamino" refers to alkyl-N(alkyl)-.

Alkyl, cycloalkyl, heterocycloalkyl, aryl, heteroaryl, aralkyl, heteroaralkyl, alkoxy, and aryloxy mentioned herein include both substituted and unsubstituted moieties. Examples of a substituent include halo, hydroxyl, amino, cyano, nitro, mercapto, alkoxycarbonyl, amido, carboxy, alkanesulfonyl, alkylcarbonyl, carbamido, carbamyl, carboxyl, thioureido, thiocyanato, sulfonamido, alkyl, alkenyl, alkynyl, alkyloxy, aryl, heteroaryl, cycloalkyl, and heterocycloalkyl, in which alkyl, alkenyl, alkynyl, alkyloxy, aryl, heteroaryl, cycloalkyl, and heterocycloalkyl may further substituted.

The term "compound", when referring to a compound of formula (I), also includes its salts, solvates, and prodrugs. A salt can be formed between an anion and a positively charged group (e.g., amino) on a compound. Examples of a suitable anion are chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. A salt can also be formed between a cation and a negatively charged group. Suitable cations include sodium ion, potassium ion, magnesium ion, calcium ion, and ammonium cation such as tetramethyl-ammonium ion. Further, a salt can contain quaternary nitrogen atoms. A solvate refers to a complex formed between an active compound and a pharmaceutically acceptable solvent. Examples of a pharmaceutically acceptable solvent include water, ethanol, isopropanol, ethyl acetate, acetic acid, and ethanolamine. A prodrug refers to a compound that, after administration, is metabolized into a pharmaceutically active drug. Examples of a prodrug include esters and other pharmaceutically acceptable derivatives.

The compounds may contain one or more non-aromatic double bonds or asymmetric centers. Each of them occurs as a racemate or a racemic mixture, a single R enantiomer, a single S enantiomer, an individual diastereomer, a diastereometric mixture, a cis-isomer, or a trans-isomer. Compounds of such isomeric forms are within the scope of this invention. They can be present as a mixture or can be isolated using chiral synthesis or chiral separation technologies.

This invention also features use of one or more of the above-described compounds of formula (I) for the manufacture of a medicament for treating and preventing a condition involving CD47 upregulation.

The term "treating" or "treatment" refers to administering one or more of the compounds of formula (I) to a subject, who has a condition involving CD47 upregulation, with the purpose to confer a therapeutic effect, e.g., to cure, relieve, alter, affect, ameliorate, or prevent the condition, its symptoms, or the predisposition toward it. "An effective amount" refers to the amount of a compound that is required to confer the therapeutic effect. Effective doses will vary, as recognized by those skilled in the art, depending on the types of symptoms treated, route of administration, excipient usage, and the possibility of co-usage with other therapeutic treatment.

To practice the method of the present invention, a composition having one or more of the above-described compounds can be administered parenterally, orally, nasally, rectally, topically, or buccally. The term "parenteral" as used herein refers to subcutaneous, intracutaneous, intravenous, intraperitoneal, intramuscular, intraarticular, intraarterial, intrasynovial, intrasternal, intrathecal, intralesional, or intracranial injection, as well as any suitable infusion technique.

A sterile injectable composition can be a solution or suspension in a non-toxic parenterally acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono- or di-glycerides). Fatty acid, such as oleic acid and its glyceride derivatives, are useful in the preparation of injectables, as are natural pharmaceutically acceptable oils, such as olive oil and castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens and Spans or other similar emulsifying agents or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A nasal aerosol or inhalation composition can be prepared according to techniques well known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents.

A composition having one or more of the above-described compounds can also be administered in the form of suppositories for rectal administration.

The carrier in the pharmaceutical composition must be "acceptable" in the sense that it is compatible with the active ingredient of the composition (and preferably, capable of stabilizing the active ingredient) and not deleterious to the subject to be treated. One or more solubilizing agents can be utilized as pharmaceutical excipients for delivery of an active compound. Examples include colloidal silicon oxide, magnesium stearate, cellulose, sodium lauryl sulfate, and D&C Yellow # 10.

The details of one or more embodiments of the invention are set forth in the description and drawings below. Other features, objects, and advantages of the invention will be apparent from the description, the drawings, and the claims.

### BRIEF DESCRIPTION OF THE DRAWING

The description below refers to the accompanying drawings:
Figure 1A shows cell surface binding of anti-human CD47-CC2C6 antibody to control (DMSO)-treated, PQ912-treated, Compound 30-treated, Compound 32-treated, or Compound 34-treated human B-cell lymphoma Raji cells, as determined by flow cytometry.
Figure 1B shows cell surface binding of anti-human CD47-CC2C6 antibody to control (DMSO)-treated, PQ912-treated, Compound 30-treated, Compound 32-treated, or Compound 34-treated human colon cancer DLD-1 cells, as determined by flow cytometry.
Figure 1C shows surface binding of anti-human CD47-CC2C6 antibody to control (DMSO)-treated, PQ912-treated, Compound 32-treated, or Compound 34-treated human ovarian cancer SK-OV-3 cells, as determined by flow cytometry.
Figures 2A shows cell surface binding of anti-human CD47-CC2C6 to control (DMSO)-treated, PQ912-treated, Compound 30-treated, Compound 32-treated, or Compound 34-treated human B-cell lymphoma Raji cells, as determined by flow cytometry.
Figures 2B shows cell surface binding of anti-human CD47-2D3 to control (DMSO)-treated, PQ912-treated, Compound 30-treated, Compound 32-treated, or Compound 34-treated human B-cell lymphoma Raji cells, as determined by flow cytometry.
Figure 3A shows cell surface binding of human SIRPα-Fc to control (DMSO)-treated, PQ912-treated, Compound 32-treated, or Compound 34-treated human B-cell lymphoma Raji cells, as determined by flow cytometry.
Figure 3B shows cell surface binding of human SIRPα-Fc to control (DMSO)-treated, PQ912-treated, Compound 32-treated, or Compound 34-treated human colon cancer DLD-1 cells, as determined by flow cytometry.
Figure 3C shows cell surface binding of human SIRPα-Fc to control (DMSO)-treated, PQ912-treated, Compound 32-treated, or Compound 34-treated human ovarian cancer SK-OV-3 cells, as determined by flow cytometry.
Figure 4A shows phagocytosis of control (DMSO)-treated, PQ912-treated, Compound 34-treated human B-cell lymphoma Raji cells with or without rituximab (i.e., an anti-human CD20 antibody) by human monocytes-derived macrophages, as determined by flow cytometry.
Figure 4B shows phagocytosis of the combination of PQ912 and rituximab-treated and the combination of Compound 34 and rituximab-treated human B-cell lymphoma Raji cells by human monocytes-derived macrophages, as determined by flow cytometry.
Figure 5A shows *in vivo* anti-tumor efficacy of control-treated, rituximab-treated, Compound 34-treated, and the combination of rituximab and Compound 34-treated human B-cell lymphoma Raji xenograft tumors, as determined by tumor volume changes.
Figure 5B shows *in vivo* anti-tumor efficacy of control-treated, rituximab-treated, Compound 34-treated, and the combination of rituximab and Compound 34-treated human B-cell lymphoma Raji xenograft tumors, as determined by body weight changes.
Figure 5C shows *in vivo* tumor growth of rituximab-treated human B-cell lymphoma Raji xenograft tumors, as determined by tumor volume changes.
Figure 5D shows *in vivo* tumor growth of the combination of rituximab and Compound 34-treated human B-cell lymphoma Raji xenograft tumors, as determined by tumor volume changes.
Figure 5E shows median survival time of control-treated, Compound 34-treated, rituximab-treated, and the combination of rituximab and Compound 34-treated human B-cell lymphoma Raji xenograft tumors.
Figure 6 shows *in vivo* anti-tumor efficacy and response rate of control-treated, rituximab-treated, and the combination of rituximab and Compound 34-treated human B-cell lymphoma Raji xenograft tumors. CR: complete regression; PR: partial regression; SD: stable disease; PD: progression disease; ORR: overall response rate.
Figure 7 shows tumor and plasma concentrations of Compound 34 in mice at hour 2, hour 4, hour 8, and hour 24 post treatment.
Figure 8A shows expression levels of CC2C6⁺ in human B-cell lymphoma Raji xenograft tumor cells treated with vehicle and Compound 34, as determined by flow cytometric analysis.
Figure 8B shows expression levels of total CD47⁺ in human B-cell lymphoma Raji xenograft tumor cells treated with vehicle and Compound 34, as determined by flow cytometric analysis.

### DETAILED DESCRIPTION

Chemotherapies utilize an anticancer drug, e.g., arsenic trioxide (Trisenox^{®}, Teva Pharmaceuticals USA, Parsippany, New Jersey), bortezomib (Velcade^{®}), carboplatin, cisplatin, cytarabine (Cytosar-U^{®} and Depocyt^{®}), eribulin, etoposide, epothilones (e.g., ixabepilone available as Ixempra^{®} from Bristol-Myers Squibb, New York, New York), hexamethylmelamine, ifosfamide (Ifex^{®}), lenalidomide, methotrexate (Trexall^{®}), oxaliplatin platinum salts, procarbazine (Matulane^{®}), taxanes (docetaxel, paclitaxel), thalidomide (Thalomid^{®}), vinblastine, vinca alkaloids, and vincristine.

These anti-cancer drugs have severe side effects, e.g., chemotherapy induced peripheral neuropathy symptoms as the drugs also attack normal cells.

Cancer cells overexpress CD47 enabling them to evade detection by the immune system and avoiding destruction by macrophages. CD47 serves as a ligand for signal regulatory protein alpha (SIRPα), which is expressed on phagocytic cells such as macrophages and dendritic cells. The interaction between CD47 and SIRPα mediates or conveys "anti-phagocytic" signals between the two cells to inhibit phagocytosis. In other words, CD47 serves as a "do-not-eat-me" signal and a marker of self. Overexpressed CD47 thus provides a possible target for cancer treatments while minimizing side effects.

Indeed, CD47 inhibitors are widely used in cancer treatments. Examples include anti-CD47 monoclonal antibodies, bispecific antibodies, and recombinant fusion protein. One drawback of the CD47 inhibitors is the so-called "antigen sink". Normal cells, having ubiquitous expression of CD47, act as "antigen sink" to soak up CD47 inhibitors. Thus, a high dose is required to achieve an effective therapeutic inhibition of CD47. When administered intravenously, the high dose of a CD47 inhibitor suppresses the "do-not-eat-me" signal on normal red blood cells, prompting macrophages to engulf them.

To address the antigen sink issue in the anti-CD47 antibody therapy, it is unexpected discovered that the compounds of formula (I) reduce the CD47-SIRPα signaling axis to activate macrophage-mediated phagocytosis, which can be further stimulated by cancer targeting antibodies via antibody-dependent cellular phagocytosis (ADCP). There are several advantages attacking the CD47-SIRPα signaling axis instead of CD47 using a compound of formula (I). First, the compounds of formula (I) do not suffer from the antigen sink problems as seen in the anti-CD47 therapy. Second, the compounds do not cause hemolysis (i.e., lysis of red blood cells) and thrombocytopenia (i.e., loss of platelets). Finally, the compounds have superior properties as compared to anti-CD47 antibodies in terms of bioavailability and delivery. These advantages are particularly beneficial for treating solid tumors.

Accordingly, the invention provides a compound of formula (I) for use in a method of treating a condition involving CD47 upregulation by administering to a subject in need thereof an effective amount of a compound of formula (I). Also within the scope is a compound of formula (I) for use in a method of increasing phagocytosis of a cell expressing CD47 by contacting the cell with a compound of formula (I) and then exposing the cell to a phagocytic cell. Still within the scope is a pharmaceutical composition containing an anti-cancer agent and a compound of formula (I).

The compounds of formula (I) described above can be prepared according to established methods. Synthetic transformations and protecting group methodologies (protection and de-protection) used for preparing these compounds are well known in the art. See, for example, R. Larock, Comprehensive Organic Transformations (3rd Ed., Wiley 2018); P. G. M. Wuts and T. W. Greene, Greene's Protective Groups in Organic Synthesis (4th Ed., John Wiley and Sons 2007); L. Fieser and M. Fieser, Fieser and Fieser's Reagents for Organic Synthesis (John Wiley and Sons 1994); L. Paquette, ed., Encyclopedia of Reagents for Organic Synthesis (2nd ed., John Wiley and Sons 2009); and G. J. Yu et al., J. Med. Chem. 2008, 51, 6044-6054.

The compounds of formula (I) can be initially screened using *in vitro* assays known in the art. They can be subsequently evaluated using *in vivo* animal models (see, e.g., Example 3 below) for their efficacy in treating a condition involving CD47 upregulation. The selected compounds can be further tested to verify their efficacy, e.g., by administering it to an animal. Based on the results, an appropriate dosage range and administration route can be determined.

The compounds of formula (I) and their pharmaceutical compositions are particularly useful for treating hematologic cancer and solid tumors involving CD47-SIRPα signaling, including cancer of the ovary, breast, colon, bladder, prostate, brain, head-and-neck, as well as hematological malignancies such as non-Hodgkin's lymphoma, chronic myeloid leukemia in blast crises, acute lymphoblastic leukemia, multiple myeloma and others. One or more of the compounds can be formulated into a pharmaceutical composition to be used alone or in combination with one or more other anti-cancer agents as described above, including therapeutic antibodies, targeted agents, chemotherapeutic agents, and immunotherapeutic agents. The compounds and pharmaceutical compositions of the invention can be used as in patients as first-line therapy or in patients who fail prior treatments. The compounds and pharmaceutical compositions are also suitable to treat other conditions involving CD47-SIRPα signaling including atherosclerosis, fibrotic diseases, and infectious diseases.

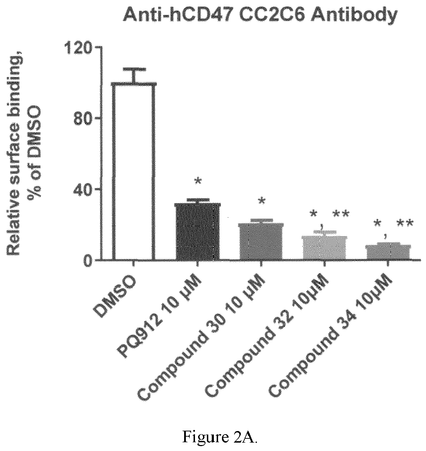

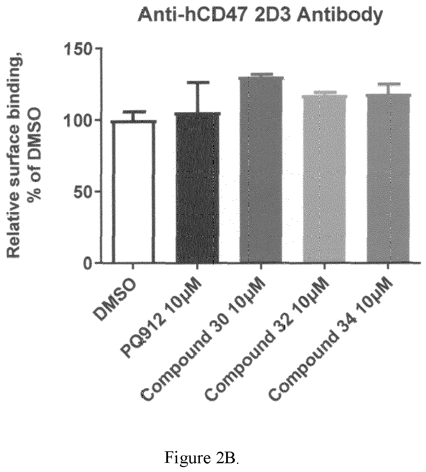

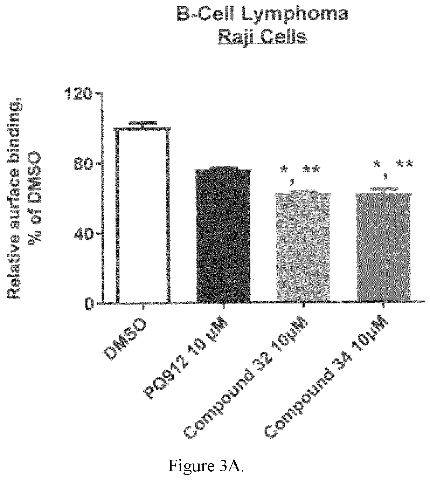

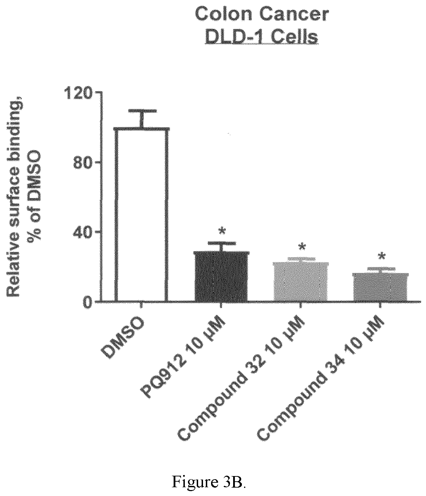

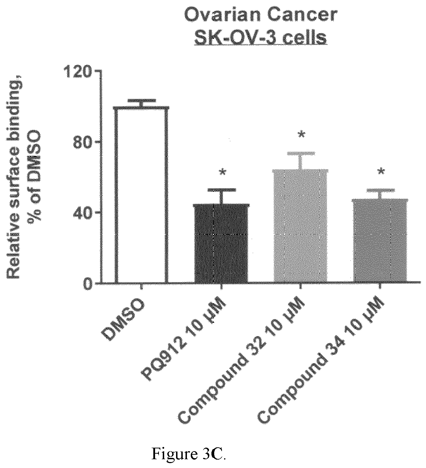

Exemplary compounds of formula (I) are described above with their structures shown. Among them, Compounds 30, 32, and 34 demonstrated particularly effective in treating cancer cells including Raji human B-cell lymphoma cell line, DLD-1 human colon cancer cell line, and SK-OV-3 human ovarian cancer cell line. Flow cytometry-based assay was used to measure effect of these compounds on binding of SIRPα and CD47. The three compounds inhibited the binding of anti-human CD47 antibody CC2C6, an antibody that recognizes CD47 and human SIRPα binding site. See
Figure 2A. On the other hand, the compounds did not inhibit the binding of anti-human CD47 antibody 2D3, an antibody that does not recognize CD47 and SIRPα binding site. See Figure 2B. The selective inhibition of the CC2C6 binding over the 2D3 binding indicates that the compounds of formula (I) treat cancer cells through inhibiting the CD47-SIRPα signaling axis. As shown in Figures 3A and 3B, compounds of formula (I) inhibiting the CD47-SIRPα-Fc binding to a greater extent than PQ912, a comparative compound having the following structure:

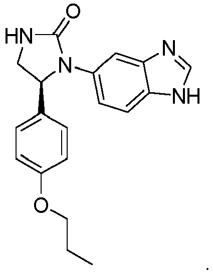

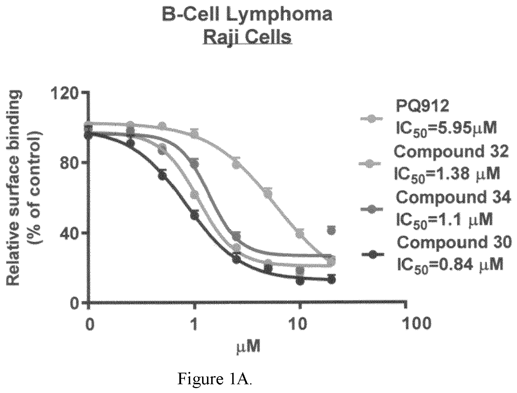

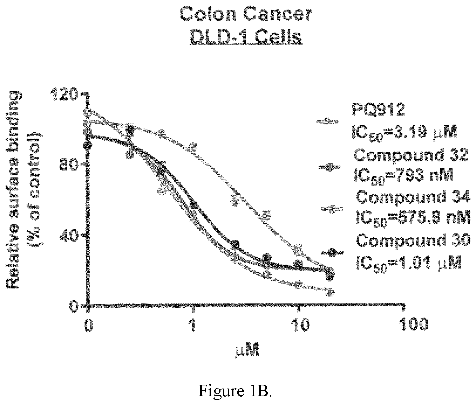

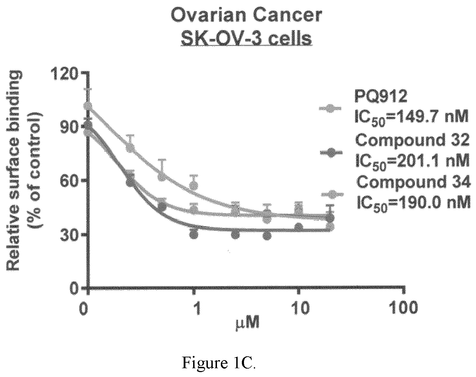

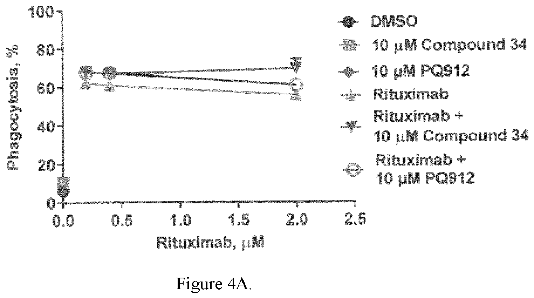

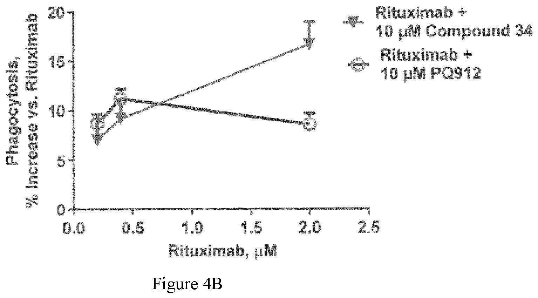

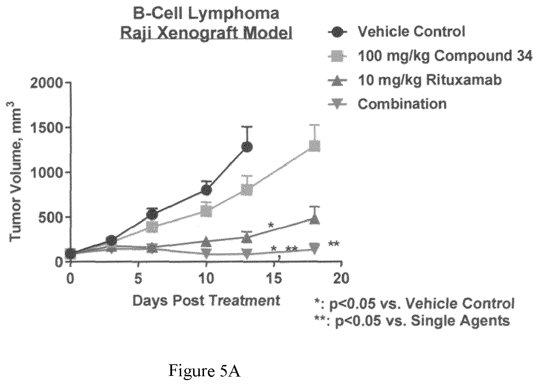

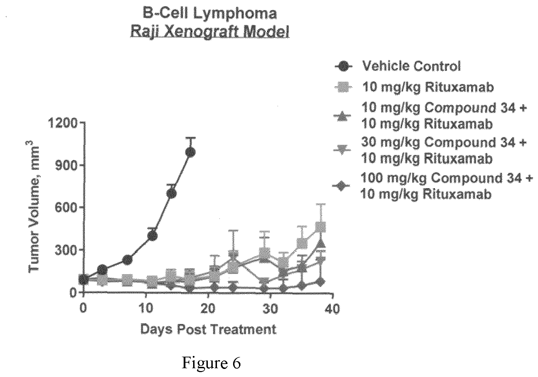

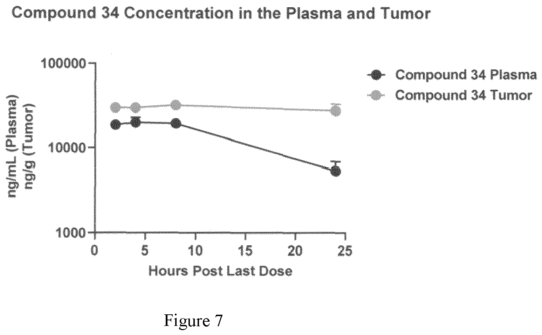

Figures 1A, 1B, and 1C show that three compounds of formula (I), i.e., Compounds 30, 32, and 34, effectively inhibited the binding of CC2C6 on the cell surface of, respectively, a human B-cell lymphoma Raji cells, human colon cancer
DLD-1 cells, and human ovarian cancer SK-OV-3 cells, indicating their effectiveness of inhibiting the CD47-SIRPα binding in the human body. Further, the concentration of 50% inhibition, IC₅₀, is at least 3-fold lower than that of Comparative Compound PQ912. See Figures 1A-1C and Table 2. As shown in Figure 4A, Compound 34 in combination with rituximab (i.e., an anti-CD20 antibody) produced 15% increase in antibody-dependent cellular phagocytosis (ADCP) as compared to rituximab alone. The ADCP of Compound 34 was confirmed by the results shown in Figure 4B. Figures 5A-5E and 6 show that the treatment using Compound 34 and rituximab produced dose-dependent tumor growth inhibition, induced tumor volume regression, and prolonged mean survival time from 28 days to 47 days as compared to the rituximab treatment, as applied to a human B-cell lymphoma xenograft model. Figure 7 shows that, within 24 hours post treatment, concentrations of Compound 34 in tumor not only were steady, but also were much higher than those in plasma, which declined drastically from hour 8 to hour 24.

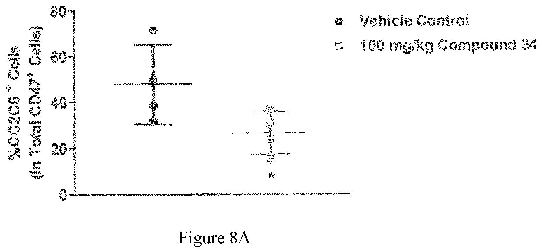

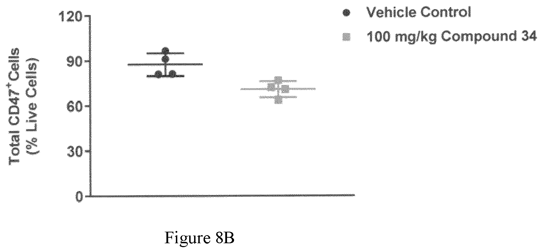

Figures 8A and 8B show that Compound 34 reduced about 56% anti-CD47 CC2C6 antibody binding on the tumor cell surface without affecting a total CD47 expression level. In addition, compounds of formula (I) exhibit excellent pharmacokinetic properties and good oral absorption in mice (%F > 50). See Table 1 below.

**Table 1**

| Compound | IV (2 mg/kg) | | PO (30 mg/kg) | | | |
|---|---|---|---|---|---|---|
| | CL | AUC Plasma | AUC Plasma | Cmax Plasma | T_{1/2} (h) Plasma | F (%) |
| PQ912 | 64 | 520 | 6359 | 2020 | 2.8 | 82 |
| 30 | 12.7 | 3037 | 21170 | 1776 | 5.2 | 47 |
| 32 | 4.0 | 8185 | 68659 | 3723 | 12.7 | 56 |
| 34 | 4.6 | 8869 | 56451 | 1983 | 9.3 | 43 |

| | | | | | | |
|---|---|---|---|---|---|---|
| Mouse pk profiles (CL: ml/min/Kg; AUC: ng/g^{∗}hr; Cmax: ng/mL) | | | | | | |

Without further elaboration, it is believed that one skilled in the art can, based on the above description, utilize the present invention to its fullest extent. The following specific examples are, therefore, to be construed as merely illustrative, and not limitative of the remainder of the disclosure in any way whatsoever.

All publications, including patent documents, cited herein are incorporated by reference in their entirety.

### EXAMPLES

### Methods of reducing CD47-SIRPα binding

Binding between CD47 and SIRPα was studied by measuring the level of CD47 binding using any suitable method. Flow cytometric analysis is useful to quantify the level of binding of CD47 to antibody CC2C6, which is then subjected to an immune-staining procedure using fluorochrome-conjugated secondary antibody followed by analysis of the immune-staining signal with a flow cytometry instrument to obtain the level of the CD47-CC2C6 binding. In another method, analysis of CD47 is performed using SIRPα-Fc formed from SIRPα fused with human IgG1, which is then subjected to an immune-staining procedure using fluorochrome-conjugated secondary antibody against human IgG1, followed by analysis of the immune-staining signal.

SIRPα is expressed on the surface of a myeloid cell such as macrophage, monocyte, neutrophil, or dendritic cell. Alternatively, SIRPα is fused with human IgG1 to form a SIRPα-Fc recombinant protein. Reduced binding between CD47 and SIRPα/SIRPα-Fc indicates that the compounds of formula (I) assist elimination of cancer cells through phagocytosis, e.g., antibody-dependent cellular phagocytosis (ADCP). An anti-cancer agent (e.g., rituximab) can be used together with a compound of formula (I). Useful anti-cancer agents are described above including anti-PD-Ll antibodies, anti-CD20 antibodies, anti-HER2 antibodies, anti-EGFR antibodies, and anti-CD47 antibodies. Other suitable anti-cancer agents are chemotherapeutic agents such as doxorubicin, epirubicin, idarubicin, methotrexate, mitoxantrone, oxaliplatin, and cyclophosphamide.

### Cell lines and culture condition

Cell lines and culture conditions used for binding experiments are listed below.

Human B-cell lymphoma Raji cells were purchased from BCRC, Taiwan. Raji cells were maintained at 37 °C under 5% CO₂ in RPMI-1640 medium (Hyclone, Marlborough, Massachusetts) supplemented with 10% fetal bovine serum (FBS) (SAFC, Sigma-Aldrich, St. Louis, Missouri) containing 2 mM L-glutamine (Hyclone, Marlborough, Massachusetts), 10 mM HEPES (Hyclone, Marlborough, Massachusetts) and 1 mM sodium pyruvate (Hyclone, Marlborough, Massachusetts).

Human colon cancer DLD-1 cells were obtained from NHRI IBPR HTS Lab, Taiwan. DLD-1 cells were maintained under the same conditions described immediately above.

SK-OV-3 cells were purchased from ATCC (Manassas, Virginia). SK-OV-3 cells were maintained at 37 °C under 5% CO₂ in McCoy's 5a medium (Gibco, ThermoFisher, Waltham, Massachusetts) with 10% fetal bovine serum (FBS) (Sigma-Aldrich, St Louis, Missouri), and 2 mM L-glutamine.

### EXAMPLE 1: Flow cytometric analysis

Antibodies and reagents used for flow cytometric analysis include:
FITC-anti-hCD47 CC2C6 (BioLegend, San Diego, California),
FITC-anti-hCD47 2D3 (eBioscience, San Diego, California),
APC-anti-human IgGFc (HP6017) (BioLegend, San Diego, California),
Human TruStain FcX (BioLegend, San Diego, California),
BV605 anti-mouse/human CD11b, clone M1/70 (BioLegend, San Diego, California),
FITC-anti-human CD68, clone Y1/82A (BioLegend, San Diego, California),
PE- anti human CD80, clone 2D10 (BioLegend, San Diego, California),
APC anti-human CD163, clone GHI/61 (BioLegend, San Diego, California),
Recombinant hSIRPα/CD172α Fc Chimera Protein (R&D System, Minneapolis, Minnesota),
7-AAD viability dye (BioLegend, San Diego, California),
pHrodo Red, SE (ThermoFisher, Waltham, Massachusetts), and
Attune^{™} NxT Flow Cytometer (ThermoFisher, Waltham, Massachusetts).

Antibody conjugates and extracellular domain of SIRPα used to analyze cell surface CD47 binding include anti-human CD47 CC2C6, 2D3, recombinant human SIRPα/CD172a Fc chimer protein, and anti-human IgG Fc. To determine inhibition of CD47 binding, cancer cells were treated with compounds of formula (I) at various concentrations for 4 days. Thereafter, the cells were washed with PBS containing 0.5% BSA (FACS buffer) followed by incubation with fluorochrome-labeled antibodies to human CD47 (i.e., CC2C6 and 2D3) at 1 : 50 dilution in FACS buffer for 30 minutes at 4 °C. The results are shown in Figures 1A-1C, 2A, and 2B. Compounds of formula (I) produced a dose-dependent inhibition of anti-CD47 CC2C6 antibody that recognized CD47 and SIRPα binding site on the tumor cell surface including B-cell lymphoma Raji cells, colon cancer cells DLD-1, and ovarian cancer SK-OV-3 cells (see Figures 1A, 1B, and 1C, respectively). Compound 34 specifically affected anti-CD47 CC2C6 antibody but not by anti-CD47 control antibody 2D3 on the tumor cell surface in Raji cells (Figures 2A and 2B). Unexpectedly, compounds of formula (I) were at least three times more potent than comparative Compound PQ912 in treating B-cell lymphoma and colon cancer (Table 2).

**Table 2**

| Compound | B-cell Lymphoma (Raji cells) IC₅₀, µM | Colon Cancer (DLD-1 cells) IC₅₀, µM | Ovarian Cancer (SK-OV3 cells) IC₅₀, µM |
|---|---|---|---|
| PQ912 | 5.95 | 3.19 | 0.15 |
| 30 | 0.84 | 1.01 | - |
| 32 | 1.38 | 0.79 | 0.2 |
| 34 | 1.1 | 0.58 | 0.19 |

To determine inhibition of the SIRPα-CD47 binding, three types of cancer cells, including B-cell lymphoma Raji cells, colon cancer DLD-1 cells, and ovarian cancer SK-OV-3 cells, were treated with compounds of formula (I) at 10 µM for 4 days followed by staining with human SIRPα/CD172a Fc chimer protein (12 µg/mL) in FACS buffer for 30 minutes at room temperature. The cells were washed twice with FACS and then stained with fluorochrome-labeled anti-human IgG Fc antibody at 1 : 50 dilution in FACS buffer for 30 minutes at 4 °C. After the indicated antibody staining, the cells were washed with FACS buffer to remove unbound antibody. Cell viability dye 7-amino-actinomycin (D7-AAD) was added to allow dead cell exclusion. Samples were analyzed by a flow cytometer (Attune^{™} NxT Flow Cytometer, ThermoFisher). The results are shown in Figures 3A-3C. Compounds of formula (I) interfered with the binding of anti-CD47 CC2C6 antibody and SIRPα-Fc chimeric protein on the tumor cell surface in B-cell lymphoma Raji cells, colon cancer DLD-1 cells, and ovarian cancer SK-OV-3 cells.

### EXAMPLE 2: Antibody-dependent cellular phagocytosis

Monocytes were isolated using EasySep^{™} direct human monocyte isolation kit per manufacture's instruction (Stem Cell Technologies, Cambridge, Massachusetts). Freshly isolated monocytes were cultured for 7 days at 37 °C and 5% CO₂ in IMDM medium (HyClone) supplemented with 25 mM HEPES, 10% FCS, 100 U/mL penicillin, 100 µg/mL streptomycin, 2 mM L-glutamine (HyClone), and 40 ng/mL human macrophage colony-stimulating factor (M-CSF, PeproTech, East Windsor, New Jersey) to allow differentiation into macrophages for 7 days. Thereafter, the cells were washed with PBS twice and cultured with 20 ng/mL recombinant human IL10 (PeproTech) for 24 hours. On the day of phagocytosis assay, culture medium was replaced with fresh IMDM medium containing 0.5% FBS. Cancer cells were cultured 4 days before the experiment in the presence of either vehicle control (DMSO), 10 µM Comparative Compound PQ912 or 10 µM compounds of formula (I). For antibody-dependent cellular phagocytosis, cancer cells were first labeled with pHrodo Red for 15 minutes at 25 °C, washed with PBS and then incubated for 3 hours in the presence or absence of rituximab and macrophages (effector-to-target cell ratio of 1:2). Thereafter, the cells were washed with PBS and detached using Accutase (Sigma-Aldrich). Internalized tumor cells by macrophages were detected as double positive phycoerythrin (PE)/Cy7-labeled anti-human CD11b and pHrodo Red cells and analyzed by a flow cytometer (Attune^{™} NxT Flow Cytometer, ThermoFisher). The results are shown in Figures 4A and 4B. Compound 34 in combination with anti-CD20 antibody rituximab produced a dose-dependent increase of ADCP compared to rituximab alone and the combination of rituximab and PQ912.

### EXAMPLE 3: In vivo experiments

Human B-cell lymphoma Raji cells were used to evaluate the efficacy of Compound 34 and rituximab in treating cancer cells. Prior to injection into animals, the cancer cells were detected as free of *Mycoplasma spp.* NOD/SCID mice (BioLasco, Taiwan) were used for *in vivo* experiments due to their higher affinity of SIRPα to human CD47. The mice used in these studies were housed in Institutional Animal Care and Use Committees-approved facility at National Health Research Institutes in accordance with guidelines for the care and use of laboratory animals.

One million Raji cells in culture medium and matrigel (1:1, v/v) were injected subcutaneously into the left flank region of the mice (n = 7 to 10 animals per group). Treatments were initiated after randomization with inclusion of tumors at approximately 100 mm³. A dosing vehicle for Compound 34 was prepared using 10% DMA : 40% PEG400 : 50% (1% CMC) (v/v/v) and was dosed orally (p.o.) at 100 mg/kg once a day, 5 days a week (Q.D.^{∗} 5). Rituximab (Roche-Genentech, South San Francisco, California) stock solution was diluted with PBS and dosed at 10 mg/kg twice a week (2QW) by intraperitoneal (i.p.) injection.

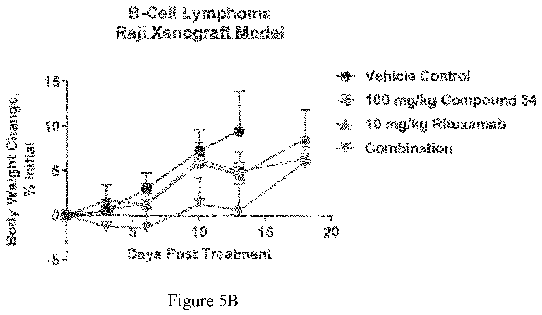

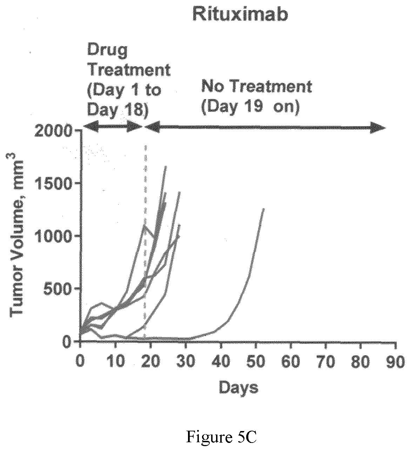

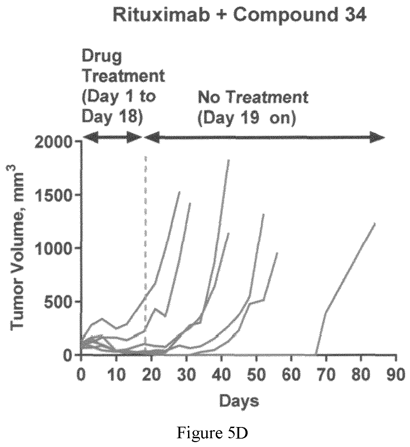

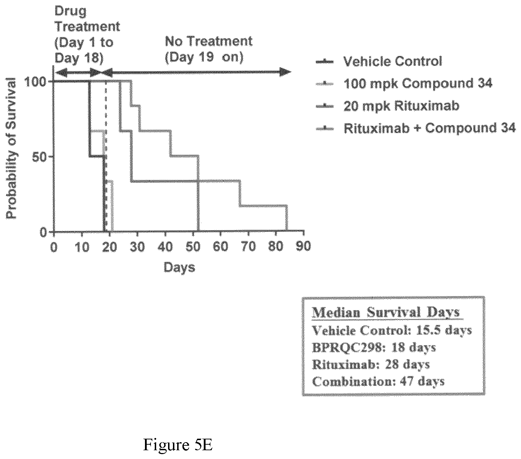

To evaluate anti-tumor efficacy and survival rates, mice were treated with vehicle control, 10 mg/kg rituximab (2QW, i.p.), 100 mg/kg Compound 34 (QD ^{∗} 5), or rituximab in combination with Compound 34. Treatment was discontinued at the end of 3 weeks. The mice were monitored for cancer growth and body weight once a week. Cancer growth was measured by volume with an electronic caliper. The volume was calculated as L × W² / 2, in which L is the length of the tumor and W is its width. Tumor size and animal body weight were measured once a week after cancer cell inoculation. Cancer response at the end of the study was calculated as tumor growth inhibition (TGI) using (1-T/C) ^{∗}100, in which T is the mean tumor volume (mm³) of the test group, and C is the mean tumor volume (mm³) of the vehicle-treated group. The results are shown in Figures 5A and 5B. Compound 34 in combination with anti-CD20 antibody rituximab produced a greater anti-tumor effect than either agent alone in human B-cell lymphoma Raji xenograft tumor model (Figure 5A). The tumor growth inhibition (TGI, % of control) by Compound 34, rituximab, and the combination were 41.4%, 78.5%, and 93.4%, respectively. No significant changes in body weight were observed in either vehicle control or treated groups during the treatment course (Figure 5B). For the survival study, treatment was discontinued at the end of 3 weeks. Thereafter, mice were followed up for tumor growth and body weight once a week. The mice were then sacrificed when tumor volume reached 2,000 mm³ or body weight loss was greater than 10%. Kaplan-Meier plots were used as the study end point to calculate percentage of animals remaining in the study in term of the time. The results are shown in Figures 5C-5E. The median survival days for vehicle control and Compound 34 were 15.5 days and 18 days, respectively (Figure 5E). Rituximab prolonged median survival days to 28 days after treatment discontinued (Figure 5C and 5E). Importantly, the combination of rituximab and Compound 34 further prolonged median survival days to 47 days (Figure 5D and 5E).

To evaluate dose-dependent anti-tumor efficacy of Compound 34 in combination with rituximab, mice were treated with vehicle control, rituximab, or rituximab in combination with Compound 34 for 6 weeks. A dosing vehicle for Compound 34 was prepared using 10% DMA : 40% PEG400 : 50% (1% CMC) (v/v/v) and was dosed orally (p.o.) at 10 mg/kg, 30 mg/kg, and 100 mg/kg once a day, 5 days a week (Q.D.^{∗} 5). Rituximab (Roche-Genentech, South San Francisco, California) stock solution was diluted with PBS and dosed at 10 mg/kg twice a week (2QW) by intraperitoneal (i.p.) injection. Cancer growth was measured by volume with an electronic caliper. The volume was calculated as L × W² / 2, in which L is the length of the tumor and W is its width. Tumor size and animal body weight were measured once a week after cancer cell inoculation. Cancer response at the end of the study was calculated as tumor growth inhibition (TGI) using (1-T/C) ^{∗}100, in which T is the mean tumor volume (mm³) of the test group, and C is the mean tumor volume (mm³) of the vehicle-treated group. The treatment response rate were classified into 4 categories: complete regression (CR), in which the tumor was undetectable at the end of study; partial regression (PR), in which the tumor volume was less than or equal to 30% of the initial tumor volume at the end of study; stable disease (SD), in which the tumor volume was between 10% to 20% of the initial tumor volume at the end of study; progression disease (PD), in which the tumor volume was greater than 50% of the initial tumor volume at the end of study. An overall response rate (ORR) is calculated based on the CR and PR groups. The results are shown in Figure 6 and Table 3 below. Compound 34 in combination with anti-CD20 antibody rituximab produced a dose-dependent growth inhibition (Figure 6) and response rates (Table 3). The tumor growth inhibition (TGI, % of rituximab) in mice treated by the combination of 10 mg/kg Compound 34 and rituximab, 30 mg/kg Compound 34 and rituximab, or 100 mg/kg Compound 34 and rituximab is 6.3%, 28.6%, and 80.5%, respectively. When treated with Compound 34 (at 100 mg/kg) in combination with rituximab, 6 out of 10 mice (60%) fell under the CR group, and 1 out of 10 mice (10%) fell under the PR group. On the other hand, no animals had CR (0%) and only 2 out of 10 mice (20%) were PR by rituximab (at 10 mg/kg), resulting in 70% ORR by the combination treatment and 20% by antibody only.

**Table 3**

| Response Rate | Rituximab (R) | 10 mg/kg 34 + 10 mg/kg R | 30 mg/kg 34 + 10 mg/kg R | 100 mg/kg 34 + 10 mg/kg R |
|---|---|---|---|---|
| CR | 0 (0%) | 1 (10%) | 2 (20%) | 6 (60%) |
| PR | 2 (20%) | 0 (0%) | 1 (10%) | 1 (10%) |
| SD | 0 (0%) | 2 (20%) | 0 (0%) | 1 (10%) |
| PD | 8 (80%) | 7 (70%) | 7 (70%) | 2 (20%) |
| ORR | 2 (20%) | 1 (10%) | 3 (30%) | 7 (70%) |

### EXAMPLE 4: Pharmacokinetic and pharmacodynamic evaluation

A pharmacokinetic study was conducted to evaluate plasma and tumor concentrations of Compound 34 using human B-cell lymphoma Raji xenograft tumors. One million Raji cells in culture medium and matrigel (1:1, v/v) were injected subcutaneously into the left flank region of each mouse. Treatments were initiated after randomization with inclusion of tumors at approximately 90 mm³. A dosing vehicle for Compound 34 was prepared using 10% DMA : 40% PEG400 : 50% (1% CMC) (v/v/v) and was dosed orally (p.o.) at 100 mg/kg once a day and 5 days a week (Q.D.^{∗} 5). The mice were treated with either the vehicle control or Compound 34 for 3 weeks. They were sacrificed 2, 4, 8, or 24 hours after the last dose (n = 4 per time point). Tumor and plasma were collected to determine concentrations of Compound 34. The results are shown in Figure 7. Compound 34 had a plasma concentration of 18,600 ng/mL 2 hours after the last dose. Its concentration was decreased to 5,357 ng/mL at 24 hours after the last dose. By contrast, Compound 34 had a tumor concentration of 29,730 ng/g tissue weight 2 hours after the last dose. In the next 22 hours, its tumor concentration remained steady at or above 27,507 ng/g tissue weight.

A pharmacodynamic study was also conducted to evaluate target engagement of Compound 34. In this study, one million Raji cells in culture medium and matrigel (1:1, v/v) were injected subcutaneously into the left flank region of each mouse. Treatments were initiated after randomization with inclusion of tumors at approximately 150 mm³. A dosing vehicle for Compound 34 was prepared using 10% DMA : 40% PEG400 : 50% (1% CMC) (v/v/v) and was dosed orally (p.o.) at 100 mg/kg once a day and 5 days a week (Q.D.^{∗} 5). The mice were treated with either the vehicle control or Compound 34 for 10 days. They were then sacrificed 24 hours after the last dose (n = 4 per group). Tumors were harvested and isolated into single cell suspensions for flow cytometry analysis. The results are shown in Figure 8A. Unexpectedly, Compound 34 reduced 56% of anti-CD47 CC2C6 antibody binding on the tumor cell surface, as indicated by a decreasing level of anti-CC2C6⁺ tumor cells in the Compound 34-treated mice as compared to the vehicle-treated mice. Figure 8B shows that Compound 34 did not affect a total CD47 expression level on the tumor cell surface.

### Statements of Invention

There is provided a method for treating a condition involving CD47 upregulation, the method comprising identifying a subject in need of treatment and administering to the subject an effective amount of a compound of formula (I): 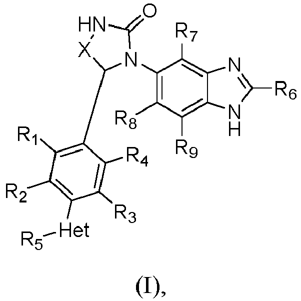 in which,
each of R₁, R₂, R₃, R₄, R₆, R₇, R₈, and R₉, independently, is H, halo, or C₁₋₆ alkyl;
R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl;
Het is a C₅₋₆ heteroaryl; and
X is CH₂ or CO.

In one aspect, each of R₁, R₂, R₃, and R₄, independently, is H or F and each of R₆, R₇, R₈, and R₉ is H.

In one aspect, R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl.

In one aspect, Het is a moiety of tetrazole, oxadiazole, thiazole, isoxazole, or thiophene.

In one aspect, each of R₁, R₂, R₃, and R₄, independently, is H or F; R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl; each of R₆, R₇, R₈, and R₉ is H; and Het is a moiety of tetrazole, oxadiazole, thiazole, isoxazole, or thiophene.

In one aspect, the compound is selected from the group consisting of Compounds 1-35.

In one aspect, the condition is a cancer, a fibrotic disease, atherosclerosis, or an infectious disease, in which the cancer is selected from cancers of the ovary, breast, colon, bladder, prostate, brain, head-and-neck, lung, stomach, pancreas, non-Hodgkin's lymphoma, chronic myeloid leukemia in blast crisis, acute lymphoblastic leukemia, and multiple myeloma; the fibrotic disease is selected from the group consisting of bladder fibrosis, heart fibrosis, idiopathic pulmonary fibrosis, kidney fibrosis, lung fibrosis, liver fibrosis, myelofibrosis, pancreas fibrosis, and scleroderma; and the infectious disease is caused by a virus, a bacterium, or a protozoan. In one aspect, the method further comprises administering to the subject an anti-cancer agent selected from the group consisting of a therapeutic antibody, a molecular targeting agent, a chemotherapeutic agent, an immuno-therapeutic agent, and combinations thereof,
wherein
the condition is a cancer;
the therapeutic antibody is selected from the group consisting of an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
the chemotherapeutic agent is selected from the group consisting of doxorubicin, epirubicin, idarubicin, methotrexate, mitoxantrone, oxaliplatin, cyclophosphamide, and combinations thereof;
the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof; and
the immuno-therapeutic agent is selected from the group consisting of nivolumab, ipilimumab, pembrolizumab, atezolizumab, avelumab, durvalumab, and combinations thereof.

In one aspect, the compound of formula (I) is administered at a dosage of 10 mg/kg to 200 mg/kg per day.

In one aspect, there is provided a method for increasing phagocytosis of a cell, comprising contacting the cell with a compound of formula (I): 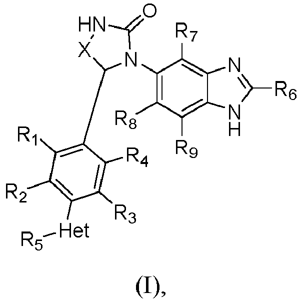 and exposing the cell to a phagocytic cell, wherein the cell expresses CD47,
wherein,
each of R₁, R₂, R₃, R₄, R₆, R₇, R₈, and R₉, independently, is H, halo, or C₁₋₆ alkyl;
R₅ is H, halo, C₁₋₆ alkyl, aralkyl, heteroaralkyl, C₃₋₁₀ cycloalkyl, or C₁₋₈ heterocycloalkyl;
Het is a C₅₋₆ heteroaryl; and
X is CH₂ or CO.

In one aspect, each of R₁, R₂, R₃, and R₄, independently, is H or F and each of R₆, R₇, R₈, and R₉ is H.

In one aspect, R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl.

In one aspect, Het is a moiety of tetrazole, oxadiazole, thiazole, isoxazole, or thiophene.

In one aspect, each of R₁, R₂, R₃, and R₄, independently, is H or F; R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl; each of R₆, R₇, R₈, and R₉ is H; and Het is a moiety of tetrazole, oxadiazole, thiazole, isoxazole, or thiophene.

In one aspect, the compound is selected from the group consisting of Compounds 1-35.

In one aspect, the cell is a cancer cell or a myeloid cell, in which the cancer cell is in the ovary, breast, colon, bladder, prostate, brain, head-and-neck, lung, stomach, pancreas, lymphatic system, bone, or blood; and the myeloid cell, having signal regulatory protein alpha (SIRPα) on its surface, is a macrophage, a monocyte, a neutrophil, a basophil, an eosinophil, or a dendritic cell.

In one aspect, the method further comprises contacting the cell with an anti-cancer agent selected from the group consisting of a therapeutic antibody, a molecular targeting agent, a chemotherapeutic agent, an immuno-therapeutic agent, and combinations thereof,
wherein
the cell is a cancer cell;
the therapeutic antibody is selected from an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
the chemotherapeutic agent is selected from the group consisting of doxorubicin, epirubicin, idarubicin, methotrexate, mitoxantrone, oxaliplatin, cyclophosphamide, and combinations thereof;
the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, and combinations thereof; and
the immuno-therapeutic agent is selected from the group consisting of nivolumab, ipilimumab, pembrolizumab, atezolizumab, avelumab, durvalumab, and combinations thereof.

In one aspect, the method further comprises contacting the cell with a CD47 inhibitor, a SIRPα inhibitor, or a combination thereof,
in which
the CD47 inhibitor is selected from the group consisting of CD47 antibodies B6H12.2, Hu5F9-G4, IT-061, CC-90002, SRF231, SHR-1603, AO-176, TJC4, TJC4-CK, SY102, PSTx-23, MBT-001, IMC-002, HMBD-004B, HLX24, BAT6004, AUR-105, AUR-104, and LYN00301, CD47 bi-specific antibodies NI-1701, IBI188, IMM03, NI-1801, PDL1/CD47 BsAv, IMM2502, IBI322, ABP-160, HMBD-004A, and BH-29xx, and combinations thereof; and
the SIRPα inhibitor is selected from the group consisting of SIRPα inhibitors ALX148, KWAR23, TTI-621, TTI-622, OSE-172, CC-95251, IMM01, FSI-189, and CTX-5861, SIRPα bi-specific inhibitors SL-172154, IMM02, and DSP107, and combinations thereof.

In one aspect, there is a provided a pharmaceutical composition comprising an anti-cancer agent and a compound of formula (I): 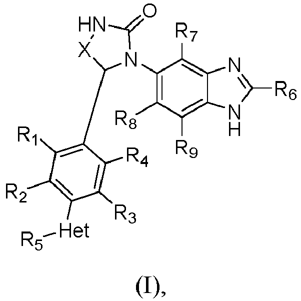 in which,
each of R₁, R₂, R₃, R₄, R₆, R₇, R₈, and R₉, independently, is H, halo, or C₁₋₆ alkyl;
R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl;
Het is a C₅₋₆ heteroaryl; and
X is CH₂ or CO.

In one aspect, each of R₁, R₂, R₃, and R₄, independently, is H or F and each of R₆, R₇, R₈, and R₉ is H.

In one aspect, R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl.

In one aspect, Het is a moiety of tetrazole, oxadiazole, thiazole, isoxazole, or thiophene.

In one aspect, each of R₁, R₂, R₃, and R₄, independently, is H or F; R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl; each of R₆, R₇, R₈, and R₉ is H; and Het is a moiety of tetrazole, oxadiazole, thiazole, isoxazole, or thiophene.

In one aspect, the compound is selected from the group consisting of Compounds 1-35.

In one aspect, the anti-cancer agent is selected from the group consisting of a therapeutic antibody, a molecular targeting agent, a chemotherapeutic agent, an immuno-therapeutic agent, and combinations thereof,
in which
the therapeutic antibody is selected from the group consisting of an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
the chemotherapeutic agent is selected from the group consisting of doxorubicin, epirubicin, idarubicin, methotrexate, mitoxantrone, oxaliplatin, cyclophosphamide, and combinations thereof;
the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof; and
the immuno-therapeutic agent is selected from the group consisting of nivolumab, ipilimumab, pembrolizumab, atezolizumab, avelumab, durvalumab, and combinations thereof.

In one aspect, there is provided a compound for use in a treatment of a condition involving CD47 upregulation or in a method of increasing phagocytosis of a cell, wherein the compound is a compound of formula (I): 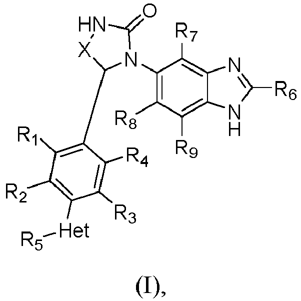
or a stereoisomer or a mixture of stereoisomers thereof, or a pharmaceutically acceptable salt, solvate, hydrate, co-crystal, clathrate, or polymorph thereof,
in which,
   each of R₁, R₂, R₃, R₄, R₆, R₇, R₈, and R₉, independently, is H, halo, or C₁₋₆ alkyl;
   R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl;
   Het is a C₅₋₆ heteroaryl; and
   X is CH₂ or CO.

In one aspect, each of R₁, R₂, R₃, and R₄, independently, is H or F and each of R₆, R₇, R₈, and R₉ is H.

In one aspect, R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl.

In one aspect, Het is a moiety of tetrazole, oxadiazole, thiazole, isoxazole, or thiophene.

In one aspect, the compound is selected from the group consisting of Compounds 1-35.

In one aspect, the condition is a cancer, a fibrotic disease, atherosclerosis, or an infectious disease, in which the cancer is selected from cancers of the ovary, breast, colon, bladder, prostate, brain, head-and-neck, lung, stomach, pancreas, non-Hodgkin's lymphoma, chronic myeloid leukemia in blast crisis, acute lymphoblastic leukemia, and multiple myeloma; the fibrotic disease is selected from the group consisting of bladder fibrosis, heart fibrosis, idiopathic pulmonary fibrosis, kidney fibrosis, lung fibrosis, liver fibrosis, myelofibrosis, pancreas fibrosis, and scleroderma; and the infectious disease is caused by a virus, a bacterium, or a protozoan.

In one aspect, the compound for use as set out above is used in combination with an anti-cancer agent selected from the group consisting of a therapeutic antibody, a molecular targeting agent, a chemotherapeutic agent, an immuno-therapeutic agent, and combinations thereof,
wherein
the condition is a cancer;
the therapeutic antibody is selected from the group consisting of an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
the chemotherapeutic agent is selected from the group consisting of doxorubicin, epirubicin, idarubicin, methotrexate, mitoxantrone, oxaliplatin, cyclophosphamide, and combinations thereof;
the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof; and
the immuno-therapeutic agent is selected from the group consisting of nivolumab, ipilimumab, pembrolizumab, atezolizumab, avelumab, durvalumab, and combinations thereof.

In one aspect, the cell is a cancer cell or a myeloid cell, in which the cancer cell is in the ovary, breast, colon, bladder, prostate, brain, head-and-neck, lung, stomach, pancreas, lymphatic system, bone, or blood; and the myeloid cell, having signal regulatory protein alpha (SIRPα) on its surface, is a macrophage, a monocyte, a neutrophil, a basophil, an eosinophil, or a dendritic cell.

In one aspect, the compound for use as set out above is used in combination with a CD47 inhibitor, a SIRPα inhibitor, or a combination thereof,
in which
the CD47 inhibitor is selected from the group consisting of CD47 antibodies B6H12.2, Hu5F9-G4, IT-061, CC-90002, SRF231, SHR-1603, AO-176, TJC4, TJC4-CK, SY102, PSTx-23, MBT-001, IMC-002, HMBD-004B, HLX24, BAT6004, AUR-105, AUR-104, and LYN00301, CD47 bi-specific antibodies NI-1701, IBI188, IMM03, NI-1801, PDL1/CD47 BsAv, IMM2502, IBI322, ABP-160, HMBD-004A, and BH-29xx, and combinations thereof; and
the SIRPα inhibitor is selected from the group consisting of SIRPα inhibitors ALX148, KWAR23, TTI-621, TTI-622, OSE-172, CC-95251, IMM01, FSI-189, and CTX-5861, SIRPα bi-specific inhibitors SL-172154, IMM02, and DSP107, and combinations thereof.

In one aspect, there is provided a pharmaceutical composition comprising an anti-cancer agent and a compound of formula (I): 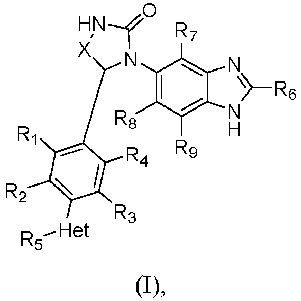
in which,
   each of R₁, R₂, R₃, R₄, R₆, R₇, R₈, and R₉, independently, is H, halo, or C₁₋₆ alkyl;
   R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl;
   Het is a C₅₋₆ heteroaryl; and
   X is CH₂ or CO,
wherein the anti-cancer agent is selected from the group consisting of a therapeutic antibody, a molecular targeting agent, a chemotherapeutic agent, an immuno-therapeutic agent, and combinations thereof,
in which
   the therapeutic antibody is selected from the group consisting of an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
   the chemotherapeutic agent is selected from the group consisting of doxorubicin, epirubicin, idarubicin, methotrexate, mitoxantrone, oxaliplatin, cyclophosphamide, and combinations thereof;
   the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof; and
   the immuno-therapeutic agent is selected from the group consisting of nivolumab, ipilimumab, pembrolizumab, atezolizumab, avelumab, durvalumab, and combinations thereof.

In one aspect, each of R₁, R₂, R₃, and R₄, independently, is H or F and each of R₆, R₇, R₈, and R₉ is H.

In one aspect, R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5-ylmethyl.

In one aspect, Het is a moiety of tetrazole, oxadiazole, thiazole, isoxazole, or thiophene.

In one aspect, the compound is selected from the group consisting of Compounds 1-35.

## Claims

1. A compound for use in a treatment of a condition involving CD47 upregulation or in a method of increasing phagocytosis of a cell, wherein the compound is a compound of formula (I): 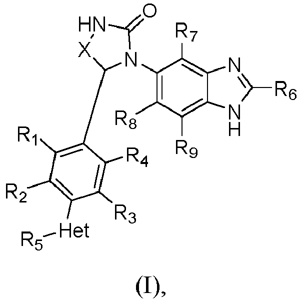
or a stereoisomer or a mixture of stereoisomers thereof, or a pharmaceutically acceptable salt, solvate, hydrate, co-crystal, clathrate, or polymorph thereof,
in which,
each of R₁, R₂, R₃, R₄, R₆, R₇, R₈, and R₉, independently, is H, halo, or C₁₋₆ alkyl;
R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl;
Het is a C₅₋₆ heteroaryl; and
X is CH₂ or CO.

2. The compound for use according to claim 1, wherein each of R₁, R₂, R₃, and R₄, independently, is H or F and each of R₆, R₇, R₈, and R₉ is H.

3. The compound for use according to claim 1 or 2, wherein R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5 -ylmethyl.

4. The compound for use according to any one of claims 1-3, wherein Het is a moiety of tetrazole, oxadiazole, thiazole, isoxazole, or thiophene.

5. The compound for use according to claim 1, wherein the compound is selected from the group consisting of Compounds 1-35.

6. The compound for use according to any one of claims 1-5, wherein the condition is a cancer, a fibrotic disease, atherosclerosis, or an infectious disease, in which the cancer is selected from cancers of the ovary, breast, colon, bladder, prostate, brain, head-and-neck, lung, stomach, pancreas, non-Hodgkin's lymphoma, chronic myeloid leukemia in blast crisis, acute lymphoblastic leukemia, and multiple myeloma; the fibrotic disease is selected from the group consisting of bladder fibrosis, heart fibrosis, idiopathic pulmonary fibrosis, kidney fibrosis, lung fibrosis, liver fibrosis, myelofibrosis, pancreas fibrosis, and scleroderma; and the infectious disease is caused by a virus, a bacterium, or a protozoan.

7. The compound for use according to any one of claims 1-6, in combination with an anti-cancer agent selected from the group consisting of a therapeutic antibody, a molecular targeting agent, a chemotherapeutic agent, an immuno-therapeutic agent, and combinations thereof,
wherein
the condition is a cancer;
the therapeutic antibody is selected from the group consisting of an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
the chemotherapeutic agent is selected from the group consisting of doxorubicin, epirubicin, idarubicin, methotrexate, mitoxantrone, oxaliplatin, cyclophosphamide, and combinations thereof;
the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof; and
the immuno-therapeutic agent is selected from the group consisting of nivolumab, ipilimumab, pembrolizumab, atezolizumab, avelumab, durvalumab, and combinations thereof.

8. The compound for use according to any one of claims 1-7, wherein the cell is a cancer cell or a myeloid cell, in which the cancer cell is in the ovary, breast, colon, bladder, prostate, brain, head-and-neck, lung, stomach, pancreas, lymphatic system, bone, or blood; and the myeloid cell, having signal regulatory protein alpha (SIRPα) on its surface, is a macrophage, a monocyte, a neutrophil, a basophil, an eosinophil, or a dendritic cell.

9. The compound for use according to any one of claims 1-8, in combination with a CD47 inhibitor, a SIRPα inhibitor, or a combination thereof,
in which
the CD47 inhibitor is selected from the group consisting of CD47 antibodies B6H12.2, Hu5F9-G4, IT-061, CC-90002, SRF231, SHR-1603, AO-176, TJC4, TJC4-CK, SY102, PSTx-23, MBT-001, IMC-002, HMBD-004B, HLX24, BAT6004, AUR-105, AUR-104, and LYN00301, CD47 bi-specific antibodies NI-1701, IBI188, IMM03, NI-1801, PDL1/CD47 BsAv, IMM2502, IBI322, ABP-160, HMBD-004A, and BH-29xx, and combinations thereof; and
the SIRPα inhibitor is selected from the group consisting of SIRPα inhibitors ALX148, KWAR23, TTI-621, TTI-622, OSE-172, CC-95251, IMM01, FSI-189, and CTX-5861, SIRPα bi-specific inhibitors SL-172154, IMM02, and DSP107, and combinations thereof.

10. A pharmaceutical composition comprising an anti-cancer agent and a compound of formula (I): 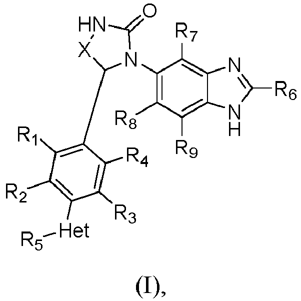
in which,
each of R₁, R₂, R₃, R₄, R₆, R₇, R₈, and R₉, independently, is H, halo, or C₁₋₆ alkyl;
R₅ is H, halo, C₁₋₆ alkyl, C₂₋₆ alkenyl, C₂₋₆ alkynyl, aralkyl, heteroaralkyl, C₃₋₁₀ cycloalkyl, or C₁₋₈ heterocycloalkyl;
Het is a C₅₋₆ heteroaryl; and
X is CH₂ or CO,
wherein the anti-cancer agent is selected from the group consisting of a therapeutic antibody, a molecular targeting agent, a chemotherapeutic agent, an immuno-therapeutic agent, and combinations thereof,
in which
the therapeutic antibody is selected from the group consisting of an anti-PD-Ll antibody, an anti-CD20 antibody, an anti-HER2 antibody, an anti-EGFR antibody, an anti-CD47 antibody, an anti-CD20-CD47 bispecific antibody, an CD19-CD47 bispecific antibody, an CD47-PD-L1 bispecific antibody, an CD47-PD-1 bispecific antibody, an anti-CD47-HER2 bispecific antibody, and combinations thereof;
the chemotherapeutic agent is selected from the group consisting of doxorubicin, epirubicin, idarubicin, methotrexate, mitoxantrone, oxaliplatin, cyclophosphamide, and combinations thereof;
the molecular targeting agent is selected from the group consisting of gefitinib, erlotinib, afatinib, dacomitinib, osimertinib, lapatinib, tucatinib, neratinib, sorafenib, regorafenib, lenvatinib, cabozentinib, sunitinib, axitinib, and combinations thereof; and
the immuno-therapeutic agent is selected from the group consisting of nivolumab, ipilimumab, pembrolizumab, atezolizumab, avelumab, durvalumab, and combinations thereof.

11. The pharmaceutical composition of claim 10, wherein each of R₁, R₂, R₃, and R₄, independently, is H or F and each of R₆, R₇, R₈, and R₉ is H.

12. The pharmaceutical composition of claim 10 or 11, wherein R₅ is H, CF₃, CH₂CF₃, methyl, ethyl, propyl, isopropyl, isobutyl, adamantanyl, propynyl, butynyl, pentynyl, cyclopropyl, cyclopropylmethyl, 4-methoxybenzyl, 4-chlorobenzyl, or 3-methylisoxazol-5 -ylmethyl.

13. The pharmaceutical composition of any one of claims 10-12, wherein Het is a moiety of tetrazole, oxadiazole, thiazole, isoxazole, or thiophene.

14. The pharmaceutical composition of any one of claims 10-13, wherein the compound is selected from the group consisting of Compounds 1-35.
